(19) Europäisches Patentamt
European Patent Office
Office européen des brevets

(11) Publication number: 0 546 261 A2

# EUROPEAN PATENT APPLICATION

(21) Application number: 92114580.1

(22) Date of filing: 27.08.92

(51) Int. Cl.5: **A61K 31/05**, A61K 31/135,
A61K 31/215, A61K 31/23,
A61K 31/365, A61K 31/40,
A61K 31/52, A61K 31/535,
A61K 31/557, A61K 31/565

(30) Priority: 09.12.91 US 758658

(43) Date of publication of application:
16.06.93 Bulletin 93/24

(84) Designated Contracting States:
AT BE CH DE DK ES FR GB GR IE IT LI LU NL
PT SE

(71) Applicant: **AMERICAN CYANAMID COMPANY**
**1937 West Main Street P.O. Box 60**
**Stamford Connecticut 06904-0060(US)**

(72) Inventor: **Ostrander, Michael Robert**
**49 Douglas Road**
**Glenridge, New Jersey 07028(US)**
Inventor: **Roizman, Bernard**
**5555 South Everett**
**Chicago, Illinois 60637(US)**
Inventor: **Gluzman, Yakov**
**24 Peach Tree Place**
**Upper Saddle River, New Jersey 07458(US)**

(74) Representative: **Wächtershäuser, Günter, Dr.**
**Tal 29**
**W-8000 München 2 (DE)**

(54) **Method of treating latent herpes viral infections.**

(57) The present invention relates to a method for treating latent viral infections in a host by administering to the host a virus-reactivating agent, to induce reactivation of the virus. This is preferably combined with administration simultaneously or subsequently an effective amount of an antiviral agent to eliminate or reduce the reactivated virus population. Viral reactivation can be induced in vivo by administration of effective amounts of compounds that activate the caicium/phosphoinositide/protein C̄ k̄īn̄āse pathway, or the adenylyl cyclase/cAMP pathway. These compounds include estrogen and estrogen analogs, phorbol esters, forskolin and forskolin derivatives, prostaglandin E and analogs, calcium ionophores, phosphodiesterase inhibitors, $\beta$-adrenergic blocking agents, and butyrates.

EP 0 546 261 A2

## BACKGROUND OF THE INVENTION

The various members of the family of viruses known as herpesviruses are causative agents of several different diseases that commonly afflict man. Two of the more familiar viruses in this family are the herpes simplex viruses (HSV) and the varicella zoster virus (VZV), both of which are associated with diseases characterized by skin lesions. Herpes simplex I is typically associated with oral and ocular lesions (fever blisters), and is spread through oral and respiratory secretions; herpes simplex II is principally associated with genital and anal lesions, and is transmitted by sexual contact. The varicella zoster virus is responsible for the production of chickenpox (varicella), primarily in children, and shingles, which primarily affects adults. Two other important herpesviruses are Epstein-Barr virus, which has been verified as the causative agent of infectious mononucleosis, and less certainly associated with "chronic fatigue syndrome", and cytomegalovirus, which causes salivary gland virus disease in newborns, but rarely causes disease in healthy adults. However, CMV subclinical infections are quite widespread, with congenital and neonatal infections, acquired from the mother before or at birth, occurring in 5-7% of live births.

Although there are some very substantial differences among the members of this family, one notable similarity is the routine occurrence of latent and recurrent infections. This is particularly apparent in HSV infections, in which initial infection may or may not result in disease; the virus persists even in the presence of high antibody titer, but remains latent, primarily in neurons, even in the absence of detectable disease. The latent virus can be activated, by causes as yet unclear, and one or more recurring episodes of disease frequently occur. Similarly, latent VZV, remaining after a childhood bout of chicken-pox, may be reactivated later in life to produce shingles in the adult. As noted above, CMV infections are primarily latent, with disease occurring rarely, although virus can be recovered from the seemingly normal salivary glands and adenoids cultured in vitro; however, this virus can also be reactivated by various causes, such as pregnancy, immunosuppression, or blood transfusion, causing a mononucleosis-like syndrome in adults. In infants, active disease can be devastating, with pneumonia and neurologic symptoms, usually resulting in death. Although recurrence of EBV-related disease is less frequently observed, the continued ability to culture affected lymphocytes of a previously afflicted but fully recovered individual indicates that latent infections of EBV are also fairly common.

The molecular basis for the recurrent herpetic infections is only partially understood (1). For example, with HSV, following primary infection, the virus multiplies at the portal of entry and infects the nerve endings enervating that site. The virus is transported to the nucleus of the sensory neuron. In the nucleus, the DNA circularizes and remains in "latent" form until activated. In the latent state, the virus is known to express only one RNA transcript the function of which is not known, but which is not associated with either the establishment or maintenance of the latent state. Because the virus does not express functions associated with its DNA synthesis, none of the drugs which inhibit viral replication have any effect on latent virus.

The events leading to the induction of latent virus and the subsequent recurrent lesion are even less well understood. Induction follows physical injury to skin at the site of initial infection, emotional stress, or hormonal imbalance. Following the induction, viral replication is activated and the virus is transported to a peripheral site at or near the site of primary infection. Few reactivations results in recurrent lesions; the physiological basis for the occurrence of lesions in some individuals and not in others is not well understood.

Studies of the process of viral reactivation, with an eye to developing methods or drugs for interfering with reactivation, have been ongoing. One study has focused on the activity of nerve growth factor (NGF in this process. NGF is known to be essential for the maintenance of sympathetic and sensory neurons of the peripheral nervous system (2, 3). Recently an ex vivo model of HSV latency in primary sympathetic neuronal cultures has been described (4-6). An essential property of this system is the requirement for NGF in the culture medium to maintain the viability of the neurons. Removal of NGF from the culture medium or use of anti-NGF antibody results in neuronal deterioration and reactivation of latent virus suggesting that deviations from normal neuronal metabolism and function are associated with the triggering event for expression of HSV. Cyclic AMP (cAMP) has been shown to antagonize some effects of NGF such as neurite formation (7-9). Also, data generated in animal models of HSV latency and reactivation show that topical application of prostaglandin E2 (10, 11) in the mouse latency model or iontophoresis of the β-adrenergic blocking agent timolol in the mouse and rabbit eye could cause reactivation of latent HSV (12, 13).

Current treatment of individuals exhibiting recurrent infections is by oral acyclovir to suppress virus replication by HSVI or II or VZV The typical duration of a preventive treatment is six weeks. Although it has been claimed that long-term treatment reduces the probability of recurrences, the common observations are that individuals who cease taking acyclovir exhibit recurrent infections at the same frequency as that observed before the treatment regimen. In addition, acyclovir is not fully effective at preventing virus replication. It has also been observed that some individuals develop recurrent infections in the face of oral acyclovir treatment.

Recurrent infections resulting from activation of latent virus can be very severe, even life-threatening, in individuals immunosuppressed by drugs or infection with another virus. Also, development of pathogenic mutants resistant to acyclovir is not uncommon. The emergence of resistance to acyclovir requires rapid development of alternative chemotherapy that is not based on nucleoside analogues. In addition, none of the experimental drugs developed to date have been shown to be effective in eliminating latent virus from model systems; new therapeutic modalities are necessary to eliminate latent virus from individuals exhibiting recurrent infections and from those at risk of serious infection because of prospective immunosuppressive therapy or with immunosuppressive diseases, such as AIDS. The present invention provides just such an alternative form of therapy.

## SUMMARY OF THE INVENTION

The present invention provides a method for treating latent viral infections by chemically controlling the recurrence of virus reactivation. Reactivation can be induced by certain classes of chemical compounds; repeated reactivation by administration of effective amounts of the reactivation agents eventually diminishes or eliminates the virus population. Controlled reactivation over a short period of time (rather than over a period of years, as occurs naturally) results in rapid elimination of recurrent episodes of disease, or else greatly reduces the frequency or severity of recurrent episodes. In a preferred embodiment, induced reactivation is accompanied by treatment of the affected host with an effective amount of an antiviral agent. Administration of the antiviral agent can be concurrent with or shortly after the induction of reactivation. The method can be used to treat any animal subject to latent virus infection, but a preferred embodiment is in treatment of human hosts for herpesvirus infection. In treatment of humans, the preferred viruses to be treated are herpes simplex virus (I and II), cytomegalovirus, Epstein-Barr virus, and varicella-zoster virus.

The invention also provides pharmaceutical compositions comprising an effective amount of an agent which can induce reactivation of a latent herpesvirus, in combination with an effective amount of an antiviral agent.

## DETAILED DESCRIPTION OF THE INVENTION

The present invention provides a means for treating latent viral infection; previously known chemotherapeutic regimens have been largely unsuccessful, primarily because the virus apparently does not express any specific function essential to establish latency, and therefore it has been impossible to design chemotherapeutic treatments that act directly on a viral function involved in the maintenance of latency. The invention relies upon the ability to induce an en masse activation of virus by chemical stimulation. The reactivation results in viral replication and transport to peripheral sites. Under these circumstances, the virus is susceptible to killing by antiviral agents which are not capable of attacking the virus in its latent stage.

A variety of different agents can be used for inducing activation of latent virus. For example, pharmacological agents that activate second messenger pathways are useful in this regard. In particular, certain agents that activate the calcium/phosphoinositide/protein C kinase pathway, or the adenylyl cyclase/cAMP pathway, have been shown to be able to activate latent virus ex vivo.

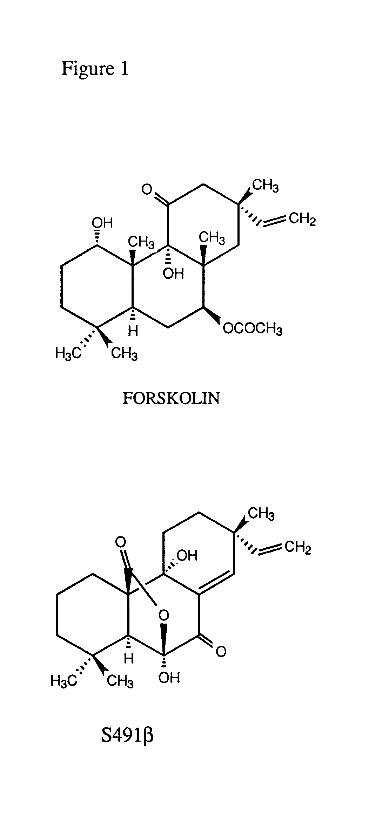

Among the preferred compounds for reactivation are prostaglandin E and related analogs, such as misoprostol, enisoprostol, rosaprostol or viprostol (14); activators of protein kinase C, such as phorbol esters (16), including, but not limited to, compounds such as 12-0-tetradecanoylphorbol-13-acetate (TPA), or diacyl glycerols, such as the compound 1,2-dioctanoylglycerol; agents that increase intracellular calcium, such as calcium agonists A23187, Bay K 8644, and CPG-28392; inhibitors of phosphodiesterase, including isobutylmethylxanthine, 2-amino pyrine (2-AP) caffeine, theophyllin, and theobromine; $\beta$-adrenergic blocking agents such as timolol, nadolol, pidolol, alprenolol, oxprenolol, or propranolol; hormones, such as estrogen, estradiol, diethylstilbestrol and mestranol; and activators of adenylate cyclase, for example, butyrates such as dibutyryl-cAMP, or forskolin and forskolin analogs, such as LL-S491$\beta$ and $\gamma$ (17). In a preferred embodiment, the forskolin derivative is LL-S491$\beta$ (Figure 1). The noted forskolin derivatives have previously been disclosed as having antiprotozoal, antibacterial and/or antiviral properties, but viral-inducing activities have not previously been recognized. Thus, the present invention also provides novel compositions comprising viral-activating effective amounts of LL-S491$\beta$ or $\gamma$, in combination with a pharmaceutically acceptable carrier.

3

The compounds are administered to individuals known to have been previously infected, and therefore likely to harbor latent infections, or to individuals not necessarily known to have been previously infected, but believed to be at high risk for infection because of, e.g., a blood transfusion or immunosuppression due to chemotherapy, a transplant, or HIV or other active viral infection. The effective amount of each drug is determined in accordance with the compound's known and safe usages. Generally, the dosage for systemic administration is about .01 mg/kg of body weight to 1000 mg/kg, with a preferred range being about .1-10 mg/kg. Mode of administration is selected according to the drug to be used and the original site of infection. Thus, administration may be topical, oral, intraocular or parenteral, i.e., intramuscular, intraperitoneal, intravenous, or subcutaneous. Useful agents can be used either alone or in combination. In some cases, because of different responsiveness of neurons, as demonstrated below in the Examples, a combination of more than one inducing agent may be most effective.

Reactivation of virus alone provides a means for reduction of viral load: when reactivation occurs, the infected neuron dies, as do the viruses associated with it. After a series of recurrences of reactivation, the number of viruses remaining is significantly reduced, as is the frequency of recurrence. This phenomenon is observed routinely, usually over a period of several years, in infected individuals who experience spontaneous, rather than controlled, reactivation. A series of controlled reactivations, on the other hand, spread over months rather than years, could alone serve to substantially reduce or eliminate the virus from the infected individual. For example, a course of treatment consisting of two to four cycles of controlled reactivation, at one-month intervals, would be effective in significantly reducing virus.

However, in order to supplement the natural progression of reactivation to elimination of virus, the reactivation cycles are preferably accompanied by treatment with an antiviral drug. The reactivation cycle takes about 3-5 days. The antiviral drug of choice can be administered simultaneously with the reactivation drug; alternately, the antiviral drug can be administered shortly after reactivation has been initiated, for example, within 24-48 hours of reactivation.

Useful antiviral compounds include, but are not limited to, acyclovir, trifluoro-thymidine (TFT), DHPG (ganciclovir) and PFA (foscarnet). The dosage range and mode of administration of antiviral compounds in the present method is determined in accordance with the dosage ranges that are employed when the compounds are used alone for antiviral therapy. These therapeutic dosages are well known to those skilled in the art.

The method of the present invention can be applied to treatment of any viral infection in which the virus undergoes a latent period from which it can be reactivated by external stimuli. A primary example of such viruses are the herpesviruses. In a preferred embodiment, the method is used to treat latent human infections, such as those caused by herpes simplex I and II, cytomegalovirus, Epstein-Barr virus, varicella zoster virus, and human herpes virus types. However, herpesviruses are also infectious agents of a wide variety of animals, particularly vertebrates, and the method is also applicable to treatment of those diseases. Other such diseases include pseudorabies virus of pigs, cytomegalovirus infections in a number of animals, such as pigs and mice, Marek's disease virus in chickens, and herpesvirus saimirii and B virus of monkeys. The foregoing list is exemplary, and not all-inclusive; those skilled in the art will readily recognize other types of herpesvirus infection to which this treatment can be applied. Moreover, the present method can be applied to treatment of other non-herpes viruses, such as papilloma viruses or human T-cell lymphotrophic viruses, which also exhibit latent phases.

The invention is further illustrated by the following non-limiting examples.

## EXAMPLES

### Example 1

Male CBA mice at six weeks of age are purchased from Jackson Labs and housed in cages at 5 mice/cage for the duration of the studies. The animals are infected with HSV-1 strain F at a dose of $10^6$ PFU/eye. The inoculation procedure is as follows: mice are anesthetized using sodium pentobarbital (0.00025 grains/mouse intraperitoneally) and corneal scarification is done using a 30 gauge needle with six vertical and six horizontal scratches; the virus inoculum is diluted to $10^8$ PFU/ml in Dulbecco's minimum essential medium (DMEM, purchased from Gibco) and 10 $\mu$l is applied to each eye. The animals are then held for a minimum of 21 days to allow for the resolution of primary ocular infection and establishment of latent virus infection. Animals are observed daily during this period.

At day 21 after infection or later, the animals are assessed for latent virus in ganglia and reactivated virus in ganglia by co-cultivation in Vero cells. Animals are sacrificed by exsanguination after being anesthetized with sodium pentobarbital as described above and both trigeminal ganglia are removed from

4

each animal using sterile forceps and each ganglion put into a separate sterile tube containing 1 ml of medium 199 (Gibco). The right trigeminal ganglion of each animal is analyzed for reactivated virus by homogenization in a sterile pestle using a tissue homogenizer. The homogenate is used to infect monolayers of Vero cells in 25 cm$^2$ flasks. Vero cells are grown in DMEM supplemented with 5% fetal bovine serum. DMEM in flasks is aspirated and replaced with 1 ml of homogenate and plates are rocked gently for 1 hour at 34$^o$C. The virus inoculum is then removed and replaced with 5 ml/flask of 199 and incubated in a humidified cell culture incubator at 37$^o$C in an atmosphere of 5% $CO_2$ and 95% air. After 72 hours the Vero monolayers are fixed by aspirating cell culture medium and replacing with 5 ml/flask of a 20% solution of Giemsa's stain for 30 minutes after removal of methanol. The stain is removed, the flasks rinsed with tap water and allowed to dry and the virus plaques are counted.

The left ganglion of each mouse is analyzed for the presence of a latent virus essentially by the protocol described for reactivated virus with the exception that intact ganglia are incubated for 5 days in a cell culture incubator prior to homogenization and co-culturing on Vero cell monolayers. This procedure allows latent virus to be expressed which can then be quantitated by co-culture.

The purpose of establishing this model of HSV latency is to use it as an in vivo test system to identify pharmacophores which would have the effect of causing reactivation of latent virus in ganglia. Two fundamental properties of this model of viral latency are the high level of latently infected animals and the relatively low level of spontaneous reactivation observed. In groups of control animals, infection of male CBA mice with an inoculum of 10$^6$ PFU/eye, latent virus is detected in 99% of the animals (n = 295/298). In those ganglia observed for level of spontaneous reactivation of virus, the observed rate over several experiments averages 3% (n = 13/416 ganglia representing 208 experimental animals).

Compounds to be tested are weighed and dissolved at the desired concentration in the appropriate solvent: dimethyl sulfoxide (DMSO), absolute ethanol or phosphate buffered saline (PBS, obtained from Gibco) to make a concentrated stock solution. Stock solutions of drug to be tested are then diluted to make working solutions which are then administered to test animals. In cases where two compounds are administered, they are administered simultaneously. Ganglia are harvested for detection of reactivated virus 4 days after pharmacological induction unless otherwise indicated.

Mestranol is administered in drinking water continuously for 5 days prior to harvesting of ganglia for quantitation of reactivated virus. Both left and right trigeminal ganglia are analyzed for reactivated virus. Mestranol is dissolved in drinking water at an appropriate concentration to achieve the daily doses as indicated based on daily water consumption of 4.5 ml/mouse/day. The control animals used for all studies is comprised of pooled untreated and placebo treated animals. Spontaneous reactivation is detected at the level of 3% (20/436 ganglia tested). The mean plaque count for control animals is 4 plaque forming units per reactivated ganglion. The median plaque count for this group of animals is 1 plaque forming unit per reactivated ganglion.

Results of the induction experiments are provided in Table 1 (mestranol alone) and Table 2 (various inducing compounds).

TABLE 1

| Induction of Latent Virus by Mestranol Administered in Drinking Water | | | | |
|---|---|---|---|---|
| Compound | Dose | Ganglia | Reactivated | Mean Plaque Count |
| Mestranol | 0.04 μg/day | 3/22 | 14% | 8 |
| | 0.12 | 1/24 | 8% | 28 |
| | 0.6 | 1/47 | 2% | 16 |
| | 1.2 | 1/30 | 3% | 38 |
| | 1.8 | 1/30 | 3% | 7 |
| | 5.4 | 3/30 | 10% | 52 |
| | 10 | 2/30 | 7% | 13 |
| | 16.2 | 2/30 | 7% | 4 |
| | 100 | 1/30 | 3% | 3 |
| | 1000 | 0/30 | - | - |

## TABLE 2

### Induction of Latent Virus with Agents
### Given by Intraperitoneal Injection

| Compound | Dose | Ganglia | Reactivated | Mean Plaque Count |
|---|---|---|---|---|
| TPA | 0.1 mg/kg | 1/16 | 6% | 1 |
| TPA + 2-AP | 0.1 mg/kg 10.0 mg/kg | 3/17 | 18% | 56 |
| TPA + Forskolin | 0.1 mg/kg 4.0 mg/kg | 2/22 | 9% | 1 |
| Forskolin | 10.0 mg/kg 20.0 mg/kg | 4/49 TOXIC | 8% | 17 |
| Mestranol + Forskolin | 0.6 mg/day 4.0 mg/kg | 4/24 | 17% | 7 |
| Mestranol + A23187 | 0.6 mg/day 4.0 mg/kg | 2/22 | 9% | 20 |
| Mestranol + A23187 | 0.6 mg/day 4.0 mg/kg | 2/22 | 9% | 20 |
| Mestranol + A23187 | 10.0 mg/day 1.0 mg/kg | 1/29 | 3% | 17 |
| Mestranol + A23187 | 10.0 mg/day 10.0 mg/kg | 5/28 | 18% | 6 |
| Mestranol + Viprostol | 10.0 mg/day 1.0 mg/kg | 5/30 | 17% | 5 |

### TABLE 2 (continued)

| Compound | Dose | Ganglia | Reactivated | Mean Plaque Count |
|---|---|---|---|---|
| Mestranol + | 10.0 mg/day | | | |
| Viprostol | 10.0 mg/kg | 3/30 | 10% | 2 |
| LL-S491$\beta$ | 4.0 mg/kg | 2/23 | 9% | 2.5 |
| | 10.0 mg/kg | 12/54 | 22% | 12 |
| | 20.0 mg/kg | 4/20 | 20% | 6 |
| | *10.0 mg/kg | 3/9 | 33% | 8 |
| | *20.0 mg/kg | 4/20 | 20% | 5 |

**Notes**:

Mestranol is administered as described above (see Table 1).

* Harvested 24 hours after induction; all others harvested at 4 days.

A number of compounds of different classes are also tested to determine if reactivation can be induced by topical application. These results are shown in Table 3.

## TABLE 3

### Induction of Latent Virus by Agents
### Given by Topical Application to the Eye

| Compound | Dose | Reactivated | Ganglia | Mean Plaque Count |
|---|---|---|---|---|
| Forskolin | 0.01% | 3/21 | 14% | 1 |
|  | 0.20% | 0/23 | - | - |
| LL-S491$\beta$ | 0.01% | 4/23 | 8% | 19 |
|  | 0.05% | 2/54 | 4% | 36 |
|  | 0.20% | 4/54 | 7% | 20 |
|  | 0.50% | 1/21 | 5% | 2 |
|  | 1.00% | 3/23 | 13% | 24 |
| Timolol | 0.01% | 5/24 | 21% | 18 |
|  | 0.02% | 2/30 | 7% | 1 |
|  | 0.10% | 3/30 | 10% | 2 |
|  | 0.20% | 4/24 | 17% | 6 |
|  | 1.00% | 2/28 | 7% | 2.5 |
| Viprostol | 0.01% | 4/22 | 18% | 5 |
|  | 0.20% | 1/24 | 5% | 5 |

**Note**:

Compound is administered topically to the eye by application of 10 $\mu$l to each eye 4 and 5 days prior to harvesting of ganglia.

Example 2

The relatively low frequency of virus reactivation in the mouse makes the use of this model for the evaluation of pharmacological inducers of latent virus less than optimal. There is an additional disadvantage in that evaluation of virus reactivation in the mouse ocular model requires that the animal be sacrificed for virus isolation as virus reactivation cannot be assessed by visual examination.

To confirm the positive results observed in the mouse in a better model, the genital infection model in the guinea pig provides an alternative for studying herpes virus latency. This model has some distinct advantages over the murine ocular infection/latency model. Firstly, the latently infected guinea pig naturally experiences spontaneous reactivations of latent virus unlike the mouse which is refractory to spontaneous reactivation of virus. This situation resembles recurrent genital infections in man and further suggests that the responsiveness of the guinea pig makes this a more appropriate system in which to study the virus reactivation and to evaluate stimuli which promote reactivation of latent virus. The second advantage of the

8

guinea pig genital infection model is that virus reactivation results in recrudescence of lesions, meaning that cutaneous lesions are expressed; again, this situation parallels that in man and provides a means of roughly quantifying virus expression. As the animals are not sacrificed, it is then possible to study the effects of repeated stimulation on the reactivation of latent virus in a stable population of test animals. For these reasons, the guinea pig model is utilized for the further examination of pharmacological induction of latent herpes simplex virus.

A number of issues can be addressed in the guinea pig experiments which could not be adequately answered in the mouse experiments. First, results are observed to determine whether the efficacy of reactivation can be increased as judged by a higher proportion of animals experiencing recrudescence or by higher mean lesion scores. Also, an attempt is made to determine whether the efficacy of reactivation is time dependent or influenced by multiple induction cycles. Finally, the effect of repetitive inductions of latent virus or diminution of reactivation efficiency or of virus load in latently infected ganglia is evaluated.

Female Hartley guinea pigs weighing approximately 250-300 grams are purchased from Charles River Laboratories. Virus infection is initiated by intravaginal administration of HSV-2 (strain G); the virus is diluted in phosphate buffered saline (PBS) at various dilutions as indicated for the individual studies described below. Diluted virus (0.8 ml) is applied to a cotton pellet which is inserted intravaginally. Drug substances to be tested are diluted in PBS to the appropriate concentration and 0.8 ml applied to a cotton pellet which is inserted intravaginally as described above. The amount of drug substance administered as well as the frequency and duration of the treatment period are indicated in Tables 4-6. Placebo treated animals received cotton pellets containing PBS alone.

Inoculum Study - The first study, an inoculum study (GP I, see Table 4), is initially to study the effects of inoculum size on primary infection and the establishment of viral latency. Animals are inoculated intravaginally with HSV-2 (G strain) at inocula of $10^5$, $10^6$, $10^7$ and $10^8$ PFU per animal. Later studies to establish induction parameters are performed using these animals.

Induction Study - The second study, an induction study (GP II, see Table 5), uses an inoculum of $10^7$ PFU per animal based on the data from the GP I study (data not shown); this provides a population of animals infected under optimal conditions ($10^7$ PFU per animal) for further evaluation.

Induction Study - The third study, another induction study (GP III, see Table 6), comprises a large group of animals (150) infected with an inoculum of $10^7$ PFU per animal. Of 99 animals which survive the initial infection, 39 are free of genital lesions and are used for induction of latent virus and 69 are found to be affected with chronic genital lesions, presumably reflecting constant shedding of virus. Only those animals which are free of lesions upon resolution of primary infection are used in virus induction studies.

## TABLE 4

Treatment:

| Compound | S491β | Viprostol | S491β | Viprostol | S491β | S491β |
|---|---|---|---|---|---|---|
| Dose | 25 ug | 30 ug/kg | 90 ug | 90 ug | 90 ug | 100 ug |
| Dose Regimen | 1x/day | 1x/day | 2x/day | 2x/day | 2x/day | 2x/day |
| Days Treated | 55-58 | 180 | 231-233 | 231-233 | 363-365 | 445-447 |

| Results: | C | T | C | T | C | T | C | T | C | T | C | T |
|---|---|---|---|---|---|---|---|---|---|---|---|---|
| % Positive | 33 | 16 | 33 | 67 | 0 | 100 | 0 | 100 | - | 67 | - | 43 |
| # Positive | 1/3 | 1/6 | 1/3 | 4/6 | 0/1 | 5/5 | 0/1 | 3/3 | - | 2/3 | 0/2 | 3/7 |
| Mean Lesion Score | 1.0 | 1.5 | 1.0 | 1.6 | NA | 1.6 | NA | 1.7 | NA | 1.5 | NA | 2.3 |

Notes:

C = Control

T = Test

NA = Not Applicable

Mean Lesion Score - Severity of genital lesions based on a numerical scale of 1 (erythema) to 4 (ulcerated lesion)

## TABLE 5

Treatment:

| Compound | Viprostol | Viprostol | S491β | S491β | S491β |
|---|---|---|---|---|---|
| Dose | 30 ug/kg | 30 ug | 90 ug | 90 ug | 90 ug |
| Dose Regimen | 1x/day | 1x/day | 2x/day | 2x/day | 2x/day |
| Days Treated | 122 | 133 | 283-285 | 365-367 | 416-418 |

| Results: | C | T | C | T | C | T | C | T | C | T |
|---|---|---|---|---|---|---|---|---|---|---|
| % Positive | 25 | 33 | 0 | 22 | 0 | 40 | NA | 17 | 0 | 0 |
| # Positive | 1/4 | 3/9 | 0/4 | 2/9 | 0/3 | 2/5 | NA | 2/12 | 0/3 | 0/9 |
| Mean Lesion Score | 1.0 | 1.5 | NA | 1.8 | NA | 1.8 | NA | 2.0 | NA | NA |

Notes:

C  = Control

T  = Test

NA = Not Applicable

Mean Lesion Score - Severity of genital lesions based on a numerical scale of 1 (erythema) to 4 (ulcerated lesion)

## TABLE 6

Treatment:

| Compound | S491β | S491β | S491β | S491β | S491β |
|---|---|---|---|---|---|
| Dose | 100 ug | 100 ug | 100 ug | 100 ug | 100 ug |
| Dose Regimen | 2x/day | 2x/day | 2x/day | 2x/day | 2x/day |
| Days Treated | 111-113 | 125-127 | 138-140 | 153-155 | 174-176 |

| Results: | C | T | C | T | C | T | C | T | C | T |
|---|---|---|---|---|---|---|---|---|---|---|
| % Positive | 60 | 77 | 0 | 79 | 0 | 62 | 0 | 54 | 40 | 79 |
| # Positive | 3/5 | 30/39 | 0/5 | 26/33 | 0/3 | 20/32 | NA | 19/35 | 2/5 | 15/19 |
| Mean Lesion Score | 2.0 | 1.8 | NA | 2.0 | NA | 1.7 | NA | 1.5 | 2.0 | 1.8 |

Notes:

C   = Control

T   = Test

NA = Not Applicable

Mean Lesion Score - Severity of genital lesions based on a numerical scale of 1 (erythema) to 4 (ulcerated lesion)

The major goals of latency reactivation studies in the guinea pig are to increase the efficiency of reactivation as judged by an increased proportion of animals expressing reactivated virus and/or a greater mean lesion score of treated animals relative to controls and to demonstrate the eradication of latent virus as evidenced by decreases of reactivation with time after induction treatment. The studies discussed above in the mouse show that pharmacological reactivation is possible, albeit at a low level. Induced reactivation in the mouse typically occurs in between 15-20% of the animals. A basic assumption underlying the application of this observation is that virus reactivation must be "efficient" in order to have the desired effect of depleting the host of latently infected neurons.

These studies in the guinea pig show that pharmacological inducers of virus reactivation as determined in the mouse model are much more efficient at inducing the reactivation of latent virus in this animal model. Thus, the guinea pig genital infection model is more relevant to the disease process in man and this animal model is believed to be more predictive of the clinical situation.

There are several points to be made with respect to the data presented. Firstly, in all three studies, the first induction event results in a relatively high proportion of placebo treated animals experiencing reactivation. One explanation of this is that populations of neurons with different levels of responsiveness to external stimuli can become latently infected; it is presumed that highly responsive neurons undergo reactivation during early inductions and are eliminated from the pool of latently infected cells. That this is the case can be inferred from the subsequent lack of observable virus reactivation in placebo treated animals in later inductions.

Another important observation is that in drug treated animals, the initial reactivation is in general modest with respect to the proportion of responding animals (Tables 4 and 5). Animals in study GP III show very efficient reactivation upon first induction in both placebo and drug treated animals; this may be the result of higher rate of establishment of latency in a population of highly responsive neurons than in studies GP I and GP II. Subsequent inductions, however, result in a greater proportion of the animals responding, suggesting some kind of sensitization phenomenon; this effect is perhaps due to a requirement for amplification of latent virus to a critical threshold which then results in reactivation. Further induction events in GP I and GP II result in fewer animals responding to the inducing agent, suggesting that latently infected neurons may have been eliminated from the host. However, this is not the case with GP III. After five inductions with S491β only, the majority of animals are still responsive. The difference between GP III and the other studies is that GP III animals are induced with S491β only, whereas animals from the other studies are induced with both S491β and Viprostol. It is surmised from these studies that there may be populations of neurons which differ in terms of pharmacological responsiveness and that treatment to achieve complete eradication of latently infected cells may involve the use of more than one inducing drug.

## CONCLUSION

This invention provides a means of treating latent viral infection in animals or man by administration of a pharmacological agent which has the capacity to cause the reactivation of the latent virus. Current antiviral therapy is useful only when virus is actively replicating and therefore these agents have no effect on latent virus. Activation of latent virus with appropriate antiviral therapy should provide a way of treating latent virus infections in vivo with the result that the latent virus in the host is dramatically reduced or eliminated.

These data are presented to support the claim that various pharmacophores administered to an animal harboring a latent virus can cause the reactivation of that virus in a predictable fashion. Such agents can be given topically or systemically by injection or ingestion. The reactivation of latent virus in the mouse is quantitated directly by excision of latently infected ganglia and culturing of ganglion homogenates on Vero cells to allow for the reactivated virus to grow. In these studies reactivation is assessed by two parameters: the relative number of ganglia expressing virus compared to controls and the amount of virus reactivated relative to controls as indicated by the mean plaque count. Spontaneous reactivation in this model is low at 3% with respect to number of reactivation events measured. Furthermore, the amount of virus produced during a spontaneous uninduced reactivation is low. The mean plaque count for control animals is determined to be 4 plaque forming units per event with the median plaque count at 1 plaque forming unit per event.

Although the mouse data presented above may, upon superficial inspection, suggest that the chosen agents are not very efficient in reactivating virus, the results must be read in view of the characteristics of the animal model employed. The mouse model of herpes virus latency is known to be refractory to spontaneous reactivation, and this observation is supported by the data presented in terms of the low rate of reactivation in control animals as well as the amount of virus induced as indicated by plaque count. In the majority of cases, the induced reactivation events results in more virus being reactivated from latency than in the control group. Although the increase in some cases appears relatively small, when interpreted in the light of the recognized difficulty in obtaining any reactivation in the mouse model, this observed increase must be interpreted as a significant biological event showing efficacy of the treatment compounds in stimulating viral reactivation.

The results observed with the guinea pig experiments further reinforce the positive observations in the mouse experiments. The pharmacological reactivation of latent virus with agents shown to be useful at low efficiency in mouse is shown to be very efficient in the guinea pig animal model, thereby confirming utility. Moreover, responsiveness initially increases with multiple cycles of induction therapy. Of particular interest is the observation that reactivation, after initial increase, decreases with continued induction, suggesting the desired eradication of latent virus. Additionally, the guinea pig data suggest that a combination of activating agents may be indicated in some circumstances.

## REFERENCES

1. **Roizman, B. and Sears, A.E.** An inquiry into the mechanisms of herpes simplex virus latency. Annu. Rev. Microbiol. 41:543-571 (1987).
2. **Greene, L.A. and Shooter, E.M.** The nerve growth factor: biochemistry, synthesis and mechanism of action. Annu. Rev. Neurosci. 3:355-402 (1980).
3. **Theonen, H. and Barde, Y-A.** Physiology of nerve growth factor. Physiol. Rev. 60:1284-1335 (1980).
4. **Wilcox, C.L. and Johnson, Jr., E.M.** Nerve growth factor deprivation results in the reactivation of latent herpes simplex virus in vitro. J. Virol. 61:2311-2315 (1987).
5. **Wilcox, C.L. and Johnson, Jr., E.M.** Characterization of nerve growth factor-dependent herpes simplex virus latency in neurons in vitro. J. Virol. 62:393-399 (1988).

6. **Wilcox, D.L., Smith, R.L., Freed, C.R. and Johnson, Jr., E.M.** Nerve growth factor-dependence of herpes simplex virus latency in peripheral sympathetic and sensory neurons in vitro. J. Neurosci 10(4)-:1268-1275 (1990).

7. **Richter-Landsberg, C. and Jastorff, B.** The role of cAMP in nerve growth factor-promoted neurite outgrowth n PC12 cells. J. Cell Biol. 102:821-829 (1986).

8. **Greene, L.A., Drexler, S.A., Connolly, J.L., Ructenstein, A. and Green S.H.** Selective inhibition of responses to nerve growth factor and of microtubule-associated protein phosphorylation by activators of adenylate cyclase. J. Cell Biol. 103:1967-1978 (1986).

9. **Doherty, P., Mann, D.A. and Walsh, F.S.** Cholera toxin and dibutyryl cyclic AMP inhibit the expression of neurofilament protein induced by nerve growth factor in cultures of naive and primed PC12 cells. J. Neurochem. 49:1696-1687 (1987).

10. **Blyth, W.A., Hill, T.J., Field, H.J. and Harbour, D.A.** Reactivation of herpes simplex virus infection by ultraviolet light and possible involvement of prostaglandins. J. gen. Virol. 33:547-550 (1976).

11. **Kurane, I., Tsuchiya, Y., Sehizawa, T. and Kumagai, K.** Inhibition by indomethacin of in vitro reactivation of latent herpes simplex virus type 1 in murine trigeminal ganglia J. gen. Virol. 65:1665-1674 (1984).

12. **Harwick, J., Romanowski, E., Araullo-Cruz and Gordon, Y.J.** Timolol promotes reactivation of latent HSV-1 in the mouse iontophoresis model. Invest. Ophthalmol. Vis. Sci. 28:580-584 (1987).

13. **Hill, J.M., Shimomura, Y., Dudley, J.B., Berman, E., Haruta, Y., Kwon, B.S. and Maguire, L.J.** Timolol induces HSV-1 ocular shedding in the latently infected rabbit. Invest. Ophthalmol. Vis. Sci. 28:585-590 (1987).

14. **Birnbaum, J.E., Cervoni, P., Chan, P.S., Chen, S-M.L., Floyd, M.B., Grudzinskas, C.V., Weiss, M. and Dessy, F.** Prostaglandins and congeners 29. (16RS)-± -15-deoxy-16-hydroxy-16-vinyl-prostaglandin E2, an orally and transdermally active hypotensive agent of prolonged duration. J. Med. Chem. 25:492-494 (1982).

15. **Seamon, K.B., Padgett, W. and Daly, J.W.** Forskolin: unique diterpene activator of adenylate cyclase in membranes and in intact cells. Proc. Nat. Acad. Sci. 78:3363-3367 (1981).

16. **zur Hausen, H., O'Neill, F.J. and Freese, U.K.** Persisting oncogenic herpesvirus induced by the tumour promoter TPA. Nature 272:373-375 (1978).

17. **Ellestad, G.A., Kunstman, M.P., Mirando, P., and Morton, G.O.** Structures of Fungal Diterpene Antibiotics LL-S491$\beta$ and $\gamma$. J Am. Chem. Soc. 94:6206-6208 (1972).

## Claims

1. A pharmaceutical composition comprising an agent for use in stimulating in vivo latent virus reactivation, wherein the agent is a compound which activates a second messenger pathway.

2. The composition of Claim 1, in which the pathway is the calcium/phosphoinositide/protein-C kinase pathway, or the adenylyl cyclase/cAMP pathway.

3. The composition of Claims 1-2, whith the compound is selected from the group consisting of of phorbol esters, prostaglandin E and analogs thereof, calcium ionophores, estrogens and analogs thereof, forskolin and analogs thereof, phosphodiesterase inhibitors, $\beta$-adrenergic blocking agents, and butyrates, or combinations thereof.

4. A pharmaceutical product comprising an agent for in vivo stimulation of latent virus and an antiviral agent as a combined preparation for simultaneous, separate or sequential use in the therapy of latent viral infection.

5. The product of Claim 4 in which the virus activating agent is a compound which stimulates a second messenger pathway.

6. The product of Claim 4 in which the pathway is the calcium/phosphoinositide/protein-C kinase pathway, or the adenylyl cyclase/cAMP pathway.

7. The product of Claim 6 in which the composition is selected from the group consisting of phorbol esters, prostaglandin E and analogs thereof, calcium ionophores, estrogens and analogs thereof, forskolin and analogs thereof, phosphodiesterase inhibitors, $\beta$-adrenergic blocking agents, and butyrates, or combinations thereof.

8. The product of any one of Claims 4-7 in whch the antiviral agent is selected from the group consisting of acyclovir, ganciclovir, foscarnet or trifluorothymidine.

9. The composition or product of any one of Claims 1-8 in which the infection is a herpesvirus infection.

10. A method of treating latent viral infection which comprises administering to a host harboring a latent virus an effective amount of a virus-activating agent, to induce reactivation of the virus, and administering simultaneously or subsequently an effective amount of an antiviral agent to eliminate or reduce the reactivated virus population.

11. A method for stimulating latent virus reactivation in a host in vivo which comprises administering to the host an effective amount of a compound which activates a second messenger pathway.

12. A method for treating a latent consecutive viral infection which comprises administrations to a host harboring a latent virus of an effective amount of a virus-activating agent to induce repeated episodes of reactivation of the virus, whereby viral load in the host is reduced after each episode of reactivation.

13. The method of Claims 10-12 in which the virus activating agent or compound is selected from the group consisting of phorbol esters, prostaglandin E and analogs thereof, calcium ionophores, estrogens and analogs thereof, forskolin and analogs thereof, phosphodiesterase inhibitors, $\beta$-adrenergic blocking agents, and butyrates, or combinations thereof.

14. The method of Claim 10 in which the activating agent is selected from the group consisting of acyclovir, ganciclovir, foscarnet or trifluorothymidine.

Figure 1

FORSKOLIN

S491β